(19) Europäisches Patentamt
European Patent Office
Office européen des brevets

(11) **EP 4 032 987 A1**

(12) **EUROPEAN PATENT APPLICATION**
published in accordance with Art. 153(4) EPC

(43) Date of publication:
**27.07.2022 Bulletin 2022/30**

(21) Application number: **20864756.0**

(22) Date of filing: **16.09.2020**

(51) International Patent Classification (IPC):
***C12Q 1/6886*** (2018.01)

(86) International application number:
**PCT/KR2020/012487**

(87) International publication number:
**WO 2021/054713 (25.03.2021 Gazette 2021/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.09.2019 KR 20190114977**

(71) Applicants:
• **Korea Advanced Institute of Science and
Technology
Guseong-dong, Yuseong-gu
Daejeon 34141 (KR)**

• **Pentamedix Co., Ltd.
Sujeong-gu, Seongnam-si, Gyeonggi-do 13449
(KR)**

(72) Inventors:
• **CHOI, Jung Kyoon
Sejong 30098 (KR)**
• **JUNG, Hyun Chul
Seoul 05503 (KR)**
• **CHO, Dae Yeon
Seongnam-si Gyeonggi-do 13540 (KR)**

(74) Representative: **Pons
Glorieta Rubén Darío 4
28010 Madrid (ES)**

(54) **METHOD FOR PREDICTING RESPONSE TO ANTI-CANCER IMMUNOTHERAPY USING DNA METHYLATION ABERRATION**

(57) Disclosed is a method for providing information for predicting a response to cancer treatment, the method including: obtaining information on a global DNA methylation level detected from a sample of a cancer patient; and evaluating a response to cancer treatment based on the information on the global DNA methylation level.

EP 4 032 987 A1

**Description**

**[Cross-Reference To Related Application(s)]**

This application claims priority to KR Patent Application No. 10-2019-0114977 filed on September 18, 2019, the content of which is hereby incorporated by reference herein.

**[Technical Field]**

The present disclosure relates to a method of providing information for predicting a response to cancer treatment using DNA methylation aberration, and a device for predicting a response to cancer treatment using DNA methylation aberration. More specifically, the present disclosure relates to a method for providing information for predicting a response to immunotherapy of cancer patients using global DNA methylation level information measured based on a LINE-1 (Long Interspersed Nuclear Element-1) factor, and a device for predicting a response to immunotherapy of cancer patients using global DNA methylation level information measured based on a LINE-1 (Long Interspersed Nuclear Element-1) factor.

**[Background Art]**

Cancer is one of the highest causes of death in the world. A cure rate thereof is often determined depending on whether or not it is diagnosed early. Thus, early diagnosis of cancer via health check-ups is important. In general, a cancer test that is widely used in health checkups is an in-blood protein tumor marker test. In addition, cancer may be identified via endoscopy, biopsy, and the like.

There are three types of abnormal mutation of genome that causes cancer. First, a part of the chromosome may be changed entirely. Second, change in a sequence of chromosome or a base sequence in which 1 to 2 sites of a base sequence changes may occur. A third cause may be due to epigenetic changes as chromatin modification (Sticker T, Catenacci DV, Seiwert TY. Molecular profiling of cancer-the future of personalized cancer medicine: a primer on cancer biology and the tools necessary to bring molecular testing to the clinic. Semin Oncol 2011 38(2): 173-85). In general, mutation in which a portion or several sites of a chromosome is modified may be a genetic cause and may occur locally in somatic cells by mutagenic factors such as radiation. An exact cause of DNA methylation aberration in cancer cells as the most representative among the epigenetic genome changes has not been identified. However, the DNA methylation aberration is thought to be caused by dietary habits or environmental factors. In particular, since such epigenetic genome changes are found a lot in genome of cancerous tissues, interest in clinical applications such as the development of diagnostic methods using the epigenetic genome changes is increasing.

DNA methylation is a phenomenon in which a methyl group ($-CH_3$) is covalently added to a 5-th carbon of a cytosine pyrimidine ring. DNA methylation plays an important role in various life phenomena such as genome imprinting and X chromosome inactivation, even in normal individual development.

In cancer tissues, following two kinds of DNA methylation phenomena appear which are different from those in normal cells: global hypomethylation across the genome and hypermethylation of CpG islands located at the gene expression control site. The hypomethylation phenomenon mainly occurs in genes and intergenic regions. This is presumed to make the chromosome unstable and cause recombination, transfer, deletion, rearrangement, and the like of chromosomes during cell division. In particular, transposons such as LINE-1 are normally methylated and expression thereof is inhibited. The transposons such as LINE-1 is expressed due to hypomethylation in cancer and is metastasized throughout the genome and thus becomes a cause of chromosomal instability. In this connection, DNA methylation aberration in cancer tissues is considered to be epigenetic. Since these epigenetic changes are maintained after cell division, hypomethylation and hypermethylation have a lasting effect on the expression of transposons and genes located around CpG islands. In fact, it is known that expressions of tumor suppressors, cell cycle regulating genes, DNA repair-related genes, and cell adhesion-related genes are suppressed by DNA methylation in cancer tissues, and thus these genes fail to function (McCabe MT, Brandes JC, Vertino PM. Cancer DNA methylation: molecular mechanisms and clinical implications. Clin Cancer Res. 2009 15(12): 3927-37). Suppressing the expression of these genes may cause the cells to proliferate abnormally, and to fail to maintain genetic stability, thereby to cause additional mutations, which plays an important role in progressing cancer.

On the other hand, unlike existing anticancer drugs that target cancer cells or cancer-related genes, immune anticancer drugs such as CTLA-4 and PD-1/PD-L1 immune checkpoint inhibitors activate the body's immune system to help immune cells attack cancer cells. It is important to select a group of patients suitable for immunotherapy as only a few patients are substantially cured compared to the high price of anticancer drugs. Knowledge of factors for predicting response to the treatment is very limited. There is no research on the correlation between immunotherapy response and DNA methylation aberration.

**[Disclosure]**

**[Technical Purpose]**

A purpose of the present disclosure is to provide a method of providing information for predicting response to cancer treatment based on global DNA methylation data measured from LINE-1 transposons obtained from patient samples.

Another purpose of the present disclosure is to provide a device for predicting response to cancer treatment based on global DNA methylation data measured from the LINE-1 transposon obtained from the patient's sample.

However, the purposes to be achieved by the present disclosure are not limited to the purposes mentioned above. Other purposes not mentioned will be clearly understood by those of ordinary skill in the art from the following description.

**[Technical Solution]**

According to an aspect of the present disclosure, a method of providing information for predicting a response to cancer treatment is provided. The method includes obtaining information on a global DNA methylation level detected from a sample of a cancer patient; and evaluating a response to cancer treatment based on the information on the global DNA methylation level.

According to an embodiment, the global DNA methylation level may be calculated as an average value of methylation levels occurring in a plurality of LINE-1 transposons.

According to an embodiment, the LINE-1 transposon may be L1HS or L1PA.

According to one embodiment, the LINE-1 transposon corresponds to an evolutionary newborn LINE-1 family, and a DNA sequence provided in the RepeatMasker database (http://www.repeatmasker.org) may be used as a criterion for selecting the evolutionary newborn LINE-1 family.

According to an embodiment, in the evaluating the response to the cancer treatment, when the global DNA methylation level is low, it may be determined that resistance to cancer treatment is high.

According to an embodiment, the cancer treatment may be immunotherapy.

According to an embodiment, the sample may be cancer tissue, whole blood, serum, saliva, sputum, cerebrospinal fluid, or urine.

According to one embodiment, the cancer may be melanoma, bladder cancer, esophageal cancer, glioma, adrenal cancer, sarcoma, thyroid cancer, colorectal cancer, prostate cancer, head and neck cancer, urinary tract cancer, stomach cancer, pancreatic cancer, liver cancer, testicular cancer, ovarian cancer, endometrial cancer, cervical cancer, brain cancer, breast cancer, kidney cancer or lung cancer.

According to another aspect of the present disclosure, a method of providing information for predicting a response to cancer treatment is provided. The method includes obtaining information on a global DNA methylation level detected from a sample of a cancer patient; obtaining information on a tumor mutation burden from the sample; and evaluating a response to cancer treatment based on the information on the global DNA methylation level and the information on the tumor mutation burden.

According to another aspect of the present disclosure, a method of providing information for predicting a response to cancer treatment is provided. The method includes obtaining information on a global DNA methylation level detected from a sample of a cancer patient; obtaining information on a tumor mutation burden from the sample; acquiring information on chromosome aneuploidy from the sample; and evaluating a response to cancer treatment based on the global DNA methylation level information, the tumor mutation burden information, and the chromosome aneuploidy information.

According to another aspect of the present disclosure, a prediction device for predicting a response to cancer treatment includes a processor, in which the processor is configured to acquire information on a global DNA methylation level detected from a patient's sample; and to evaluate the response to the cancer treatment based on the information on the global DNA methylation level.

According to another aspect of the present disclosure, a prediction device for predicting a response to cancer treatment includes a processor, in which the processor is configured to acquire information on the global DNA methylation level detected from the patient's sample; obtain information on the tumor mutation burden from the sample; and evaluate the response to cancer treatment based on the information on the global DNA methylation level and the information on the tumor mutation burden.

According to another aspect of the present disclosure, a prediction device for predicting a response to cancer treatment includes a processor, in which the processor is configured to acquire information on the global DNA methylation level detected from the patient's sample; obtain information on the tumor mutation burden from the sample; obtain information on chromosome aneuploidy from the sample; and evaluate the response to cancer treatment based on the information on the global DNA methylation level, the tumor mutation burden, and the chromosome aneuploidy.

[Advantageous Effects]

The information provision method and the prediction device for predicting the response to cancer treatment according to the present disclosure may predict the resistance to the cancer treatment for the patient, based on the global DNA methylation information, tumor mutation burden information, chromosome aneuploidy information, or a combination thereof of the cancer patient, and thus may provide information related to cancer treatment response quickly and simply at high reliability.

Therefore, the information provision method and the prediction device for predicting the response to cancer treatment according to the present disclosure may be effectively used to select a patient group predicted to have good treatment effect and prognosis before proceeding with the cancer treatment, especially immunotherapy.

However, the effect of the present disclosure is not limited to the above effect. It is to be understood that the present disclosure includes all effects derived from the detailed description or a configuration of the disclosure as described in the claims.

**[Description of Drawings]**

FIG. 1 shows the correlations between cell proliferation markers (a), tumor mutation burden (b), chromosome aneuploidy level (c), and tumor infiltrating CD8+ T cell markers (d) and global DNA methylation levels for 21 types of cancers.

FIG. 2A and FIG. 2B show that global hypomethylation is associated with decreased immunity in cancer regardless of association thereof with tumor mutation burden or chromosome aneuploidy. FIG. 2A shows that the global methylation level has a positive correlation (red heatmap) with the activity of various immune cells infiltrating cancer tissues. FIG. 2B shows that the global methylation level has a positive correlation with the hallmark immune gene set expressed in cancer cells (red heatmap), and has a negative correlation with the hallmark proliferation gene set (blue heatmap).

FIG. 3A shows how the expression levels of genes in the region where DNA replication occurs late (late-replicating region) differs between patient samples with high global methylation and patient samples with low global methylation, for various types of cancers. FIG. 3A shows that in several carcinomas, low gene expression may be identified in patients with low global methylation.

FIG. 3B shows comparison of the number of CpG islands in the promoters of genes in the late-replicating region as hypermethylated compared to normal cells, between patient samples with high global methylation and patient samples with low global methylation, for various types of cancers. Based on a result of the comparison, it may be identified that CpG island hypermethylation occurs at a higher level in patients with low global methylation in several carcinomas.

FIG. 3C statistically shows that genes related to cell division are more concentrated in regions that DNA replication occurs early (early-replicating region), whereas genes related to immune response are concentrated in late-replicating regions.

FIG. 4A shows the DNA methylation pattern according to the global methylation level of the lung cancer cohort sample (n = 141) in CpG island (CGI), shore, shelf, and open sea sites.

FIG. 4B shows how methylation, tumor mutation burden, and chromosome aneuploidy levels in the CGI and open sea region vary quantitatively based on the global methylation level in the samples.

FIG. 5A shows the survival rate based on the global methylation level as analyzed using the Cox proportional hazard model in lung cancer cohort samples, and shows the results for each of the combined cohort, IDIBELL cohort, and SMC cohort.

FIG. 5B shows the survival rate based on the tumor mutation burden as analyzed using the Cox proportional risk model in the lung cancer cohort sample, and shows the results for each of the combined cohort, IDIBELL cohort, and SMC cohort.

FIG. 6 shows the comparison of survival rates based on the global methylation level and tumor mutation burden as analyzed using the Cox proportional risk model in melanoma cohort samples.

[Best Mode]

The present disclosure relates to a method for predicting response to anticancer immunotherapy using DNA methylation aberration. More specifically, a method of providing information for predicting a response to cancer treatment includes acquiring information on the global DNA methylation level detected from a sample of a cancer patient, and evaluating a response to cancer treatment based on the information on the global DNA methylation level. Further, the present disclosure relates to a device for predicting a response to cancer treatment evaluating the response to the cancer treatment based on the information on the global DNA methylation level.

Hereinafter, embodiments will be described in detail with reference to the accompanying drawings. However, various changes may be made to the embodiments, and thus the scope of the patent application is not limited or limited by these embodiments. It should be understood that all changes, equivalents, or substitutes to the embodiments are included in the scope of the rights.

The terms used in the embodiments are used for illustrative purposes only and should not be interpreted as limiting. Singular expressions include plural expressions unless the context clearly indicates otherwise. In the present specification, terms such as "comprise" or "have" are intended to designate the presence of features, numbers, steps, actions, components, parts, or combinations thereof described in the specification, but one or more other features. It is to be understood that the presence or addition of elements, numbers, steps, actions, components, parts, or combinations thereof, does not preclude in advance the possibility.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The same reference numbers in different figures represent the same or similar elements, and as such perform similar functionality. Further, descriptions and details of well-known steps and elements are omitted for simplicity of the description. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

The present disclosure was completed by discovering that global DNA methylation is related to the immune evasion response of mutated tumor and resistance to immunotherapy thereof. Thus, the present disclosure intends to provide the use of the global DNA methylation as a novel marker that may be used to predict cancer treatment response and prognosis.

According to an aspect of the present disclosure, a method of providing information for predicting a response to cancer treatment is provided. The method includes obtaining information on a global DNA methylation level detected from a sample of a cancer patient; and evaluating a response to cancer treatment based on the information on the global DNA methylation level.

In the present disclosure, the term "method for providing information for predicting response to cancer treatment" may refer to a preliminary step for diagnosis, and provides objective basic information necessary for diagnosis of cancer, and excludes clinical judgments or opinions of doctors.

In the present disclosure, the term "methylation" refers to a phenomenon in which a methyl group ($-CH_3$) is covalently coupled to the 5-th carbon of the cytosine pyrimidine ring constituting DNA. More specifically, the term "global DNA methylation" as used in the present disclosure refers to methylation occurring in the cytosine of the LINE-1 CpG site. When methylation occurs, the binding of transposons is interrupted accordingly, thereby inhibiting specific gene expression. Conversely, when demethylation or hypomethylation occurs, specific gene expression may increase.

In the present disclosure, the global DNA methylation level means the average value of methylation levels occurring in a plurality of LINE-1 transposons. The LINE-1 transposon may be L1HS or L1PA belonging to the evolutionary newborn LINE-1 family.

Further, the average value of the methylation may be measured using a microarray, methylation-specific polymerase chain reaction (MSP), real time methylation-specific polymerase chain reaction (PCR), PCR using methylation DNA-specific binding protein, pyro-sequencing, determination of absence or presence of methylation using MS-HRM (Methylation-Sensitive High-resolution Melting Curve Analysis) and methylation-sensitive restriction enzymes, DNA chip and automatic base analysis such as bisulfite sequencing. However, the disclosure is not limited thereto.

A methylation site may be classified into shelf, shore, and open sea based on the distance from CGI. When the site is within 2 kb from CGI, the site is referred to as shore. When the site is within 2 kb from shore, the site is referred to as shelf. When the site is away from CGI by 4 kb or greater, the site is referred to as open sea.

In the present disclosure, in the step of evaluating the response to the cancer treatment, when the global DNA methylation level is low, it may be determined that resistance to cancer treatment is high. Specifically, this is because the lower the global DNA methylation level, the greater inhibited the activity of immune cells infiltrating cancer tissues, such that the expression level of immune response genes expressed in cancer cells is reduced. Further, as shown in the following Examples, the survival rate of immunotherapy applied cohort cancer patients is statistically significantly reduced. The global DNA methylation level may act as a variable that acts independently of tumor mutation burden and chromosome aneuploidy information. In fact, it is identified that the global DNA methylation level is consistently exhibited in several cohorts at a higher accuracy than the tumor mutation burden as a widely used prediction factor has. Thus, using the method according to the present disclosure, the response to cancer treatment may be predicted very effectively.

The cancer treatment may be an immunotherapy, and may include an immune checkpoint inhibitor, an immune cell therapy, a therapeutic antibody, or the like. The immune checkpoint inhibitor refers to a drug that attacks cancer cells by activating T cells by blocking the activation of immune checkpoint proteins involved in T cell suppression, and may be CTLA-4, PD-1, PD-L1 inhibitor, etc., but is not limited thereto.

In the present disclosure, the sample may be cancer tissue, whole blood, serum, saliva, sputum, cerebrospinal fluid, or urine, but is not limited thereto.

Further, the cancer may be melanoma, bladder cancer, esophageal cancer, glioma, adrenal cancer, sarcoma, thyroid cancer, colorectal cancer, prostate cancer, head and neck cancer, urinary tract cancer, stomach cancer, pancreatic cancer, liver cancer, testicular cancer, ovarian cancer, endometrial cancer, cervical cancer, brain cancer, breast cancer, kidney cancer or lung cancer, but is not limited thereto.

According to another aspect of the present disclosure, a method of providing information for predicting a response to cancer treatment is provided. The method includes obtaining information on a global DNA methylation level detected from a sample of a cancer patient; obtaining information on a tumor mutation burden from the sample; and evaluating a response to cancer treatment based on the information on the global DNA methylation level and the information on the tumor mutation burden.

According to another aspect of the present disclosure, a method of providing information for predicting a response to cancer treatment is provided. The method includes obtaining information on a global DNA methylation level detected from a sample of a cancer patient; obtaining information on a tumor mutation burden from the sample; acquiring information on chromosome aneuploidy from the sample; and evaluating a response to cancer treatment based on the global DNA methylation level information, the tumor mutation burden information, and the chromosome aneuploidy information.

In the above information provision method, the descriptions of the measurement of the global DNA methylation level, the LINE-1 transposon, the cancer treatment, the sample and the carcinoma are as described above.

Further, in the present disclosure, the term "tumor mutation burden (TMB)" is a numerical value that quantitatively expresses the number of mutations, and refers to the number of mutations observed per mega base in the sequencing results of cancer tissues.

In the present disclosure, the term "chromosome aneuploidy" refers to a state in which the number of chromosomes per cell in a cell, individual or lineage is not an integral multiple of a basic number, and is larger or smaller than the integer multiple by 1 or greater, that is, refers to a state in which the cell has a genome with an incomplete composition. In the present disclosure, the chromosome aneuploidy value may be derived from CNV (Copy number variations), which may be obtained by applying a threshold of $\pm 0.2$ (mean value of LUAD and LUSC) to the $\log_2$ ratio (tumor versus normal), detecting duplicates/deletions affecting at least 10% of the chromosomal arm or 5% of the chromosome, and calculating a total sum of an absolute segment $\log_2$ ratio thereof.

According to another aspect of the present disclosure, a prediction device for predicting a response to cancer treatment includes a processor, in which the processor is configured to acquire information on a global DNA methylation level detected from a patient's sample; and to evaluate the response to the cancer treatment based on the information on the global DNA methylation level.

According to another aspect of the present disclosure, a prediction device for predicting a response to cancer treatment includes a processor, in which the processor is configured to acquire information on the global DNA methylation level detected from the patient's sample; obtain information on the tumor mutation burden from the sample; and evaluate the response to cancer treatment based on the information on the global DNA methylation level and the information on the tumor mutation burden.

According to another aspect of the present disclosure, a prediction device for predicting a response to cancer treatment includes a processor, in which the processor is configured to acquire information on the global DNA methylation level detected from the patient's sample; obtain information on the tumor mutation burden from the sample; obtain information on chromosome aneuploidy from the sample; and evaluate the response to cancer treatment based on the information on the global DNA methylation level, the tumor mutation burden, and the chromosome aneuploidy.

In the device for predicting a response to cancer treatment, the measurement of the global DNA methylation level, the LINE-1 factor, the cancer treatment, the sample and the description of the carcinoma are as described above.

Hereinafter, preferred embodiments are presented to aid in understanding the present invention. However, the following examples are provided for easier understanding of the present invention, and the contents of the present invention are not limited by the examples.

**Example 1. Derivation of correlation between global DNA methylation and immune evasion**

1-1. Selection of target data

For analysis, DNA methylation (based on Infinium Methylation 450k technology), mRNA expression and gene mutation data generated by The Cancer Genome Atlas (TCGA) were downloaded (https://gdc.cancer.gov/about-data/publications/pancanatlas). As cancer types of more than 100 patient samples with all molecular data and age information, following 21 types of cancers were selected: bladder cancer (BLCA), breast adenocarcinoma (BRCA), cervical cancer, squamous cell carcinoma, cervical adenocarcinoma (CESC), colorectal cancer (CRC), esophageal cancer (ESCA), squamous cell carcinoma of the head and neck (HNSC), clear cell type renal cell carcinoma (KIRC), papillary renal cell carcinoma (KIRP), low-grade glioma (LGG), hepatocellular carcinoma (LIHC), lung adenocarcinoma (LUAD), lung squamous cell carcinoma (LUSC), pancreatic cancer (PAAD), adrenal cancer (PCPG), prostate cancer (PRAD), sarcoma (SARC), cutaneous melanoma (SKCM), gastric adenocarcinoma (STAD), testicular cancer (TGCT), thyroid cancer (THCA), and endometrial cancer (UCEC). Those included 6968 samples. The chromosome aneuploidy level was obtained from a chart of Taylor et al. (Cancer Cell 33, 676-689.e3 (2018)).

1-2. Measurement of global methylation level

In order to measure the global methylation level, each probe was selected in which more than 90% (45 bp) of each probe sequence of the Infinium Methylation 450k microarray maps with the young LINE-1 subfamily (L1HS and L1PA) provided from the RepeatMasker database (www.repeatmasker.org). The average beta values of the probes selected from the tumor samples were averaged and used as an estimate of the global methylation level.

**Example 2. Derivation of global DNA methylation level correlation**

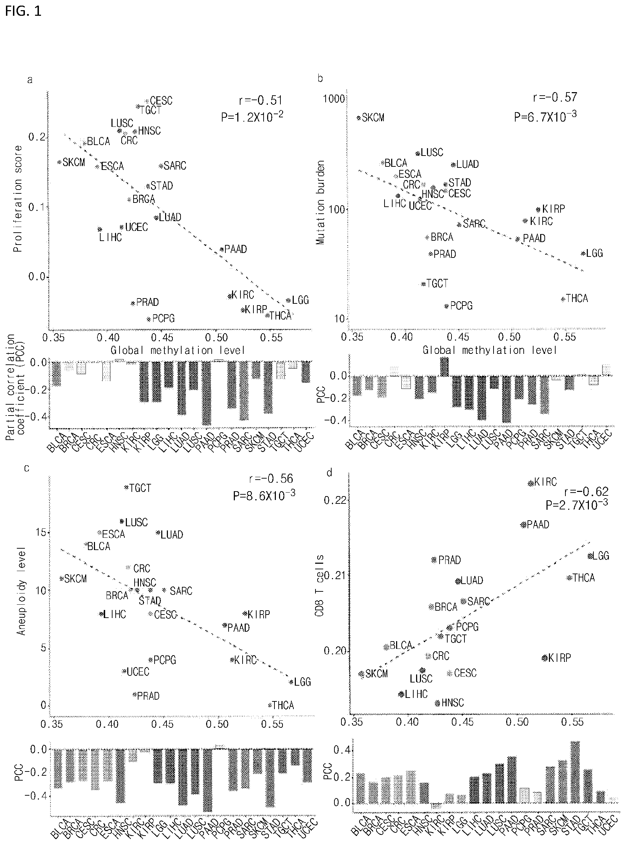

Using the values obtained in Example 1 above, correlations between global DNA methylation level and cell proliferation marker, tumor mutation burden, chromosome aneuploidy, and tumor infiltrating CD8+ T cell markers for the 21 types of cancers were derived, and the results are shown in FIG. 1. The cell proliferation markers refer to genes involved in cell division and cell cycle. The CD8+ T cell markers refer to genes specifically expressed in CD8+ T cells. The amount of expression of these genes in the given cancer tissue transcript data was determined as the marker activity. The definition of each marker followed the existing literature (Immunity 48, 812-830.e14 (2018)). The median for each cancer type was found and statistical significance was verified using Separman's correlation.

As may be seen in FIG. 1, as DNA methylation level increased, cell proliferation marker expression, tumor mutation burden and chromosome aneuploidy tended to decrease, and tumor-infiltrating CD8+ T cell marker expression tended to increase. Thus, the existing fact was identified that there was a positive correlation in which as cell division occurs, DNA methylation level gradually decreases (demethylation or methylation loss), and thus mutation of the DNA such as tumor mutation burden and chromosome aneuploidy increases. However, a new fact identified in the present disclosure is that there is a correlation between these indicators and the immune response.

In order to solve the entangled correlation, a linear regression model was applied while the mRNA expression level for each gene for each cancer type was used as a response variable, and the global methylation, tumor mutation burden, chromosome aneuploidy, tumor purity, age and tumor stage were used as a prediction variable.

A regression model using a following formula was created using an lm function of R:

$$\text{mRNA expression of gene Y} \sim \beta1^*\text{global methylation level} + \beta2^*\text{tumor mutation}$$

$$\text{burden} + \beta3^*\text{chromosome aneuploidy level} + \beta4^*\text{tumor purity} + \beta5^*\text{age} + \beta6^*\text{tumor stage}$$

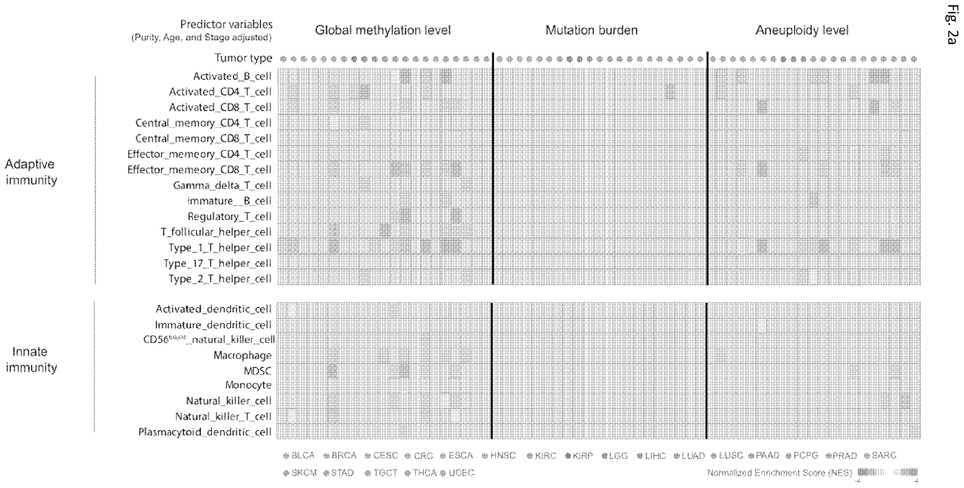

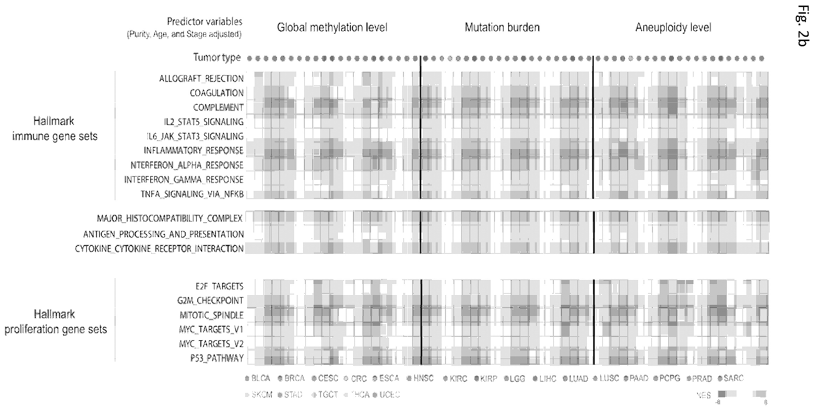

To determine the function of genes (Benjamini and Hochberg FDR < 0.05) with significant regression coefficients for three prediction variables (global methylation level, tumor mutation burden, and chromosome aneuploidy) for each cancer type, Gene Set Enrichment Analysis (GSEA; Subramanian, A. et al., Proc. Natl. Acad. Sci. 102, 15545-15550 (2005)) was executed. Cells with significant NES (Normalized Enrichment Scores) (FER < 0.25) were color-adjusted and GSEA NESs heatmaps are shown in FIG. 2A and FIG. 2B.

As a result, it was identified as shown in FIG. 2A and FIG. 2B that as the global methylation level was lower, the activity of various immune cells infiltrating the cancer tissues was lower, independently of tumor mutation burden and chromosome aneuploidy, and thus the level of expression of immune genes expressed in cancer cells was lowered.

**Example 3. Relationship between global DNA methylation and immune gene expression**

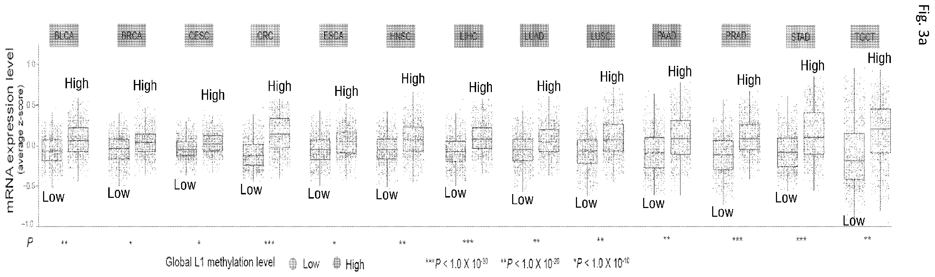

As shown in Example 2, as cell division occurs, the level of DNA methylation gradually decreases, and accordingly, the expression level of immune genes expressed in cancer cells decreases. It is known that the decrease of methyl due to cell division mainly occurs in the late-DNA replicating region. In fact, the difference in gene expression levels in regions where DNA replication occurs late between patient samples with high global methylation and patient samples with low global methylation was analyzed for each carcinoma. As a result, as shown in FIG. 3A, it was identified that the gene expression levels in patients with low global methylation were low in several carcinomas.

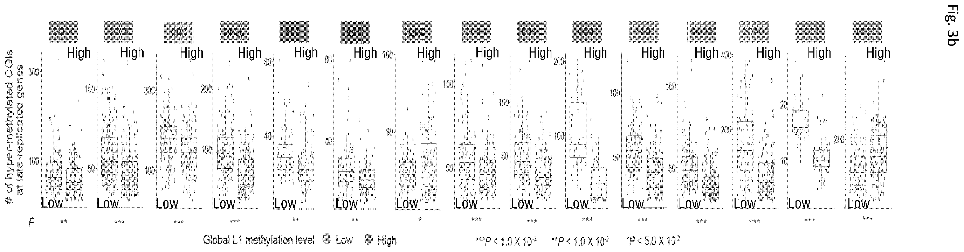

Further, it is known that the overall change in methylation of cancer occurs such that hypomethylation occurs in transposon such as LINE, while hypermethylation occurs in CpG islands present in gene promoters. In fact, the level of hypermethylation of CpG islands present in the promoters of genes in regions where DNA replication occurs late was compared between patient samples with high global methylation and patient samples with low global methylation, for each carcinoma. As a result, as shown in FIG. 3B, it was identified that CpG island hypermethylation occurred at a higher level in patients with low global methylation in several carcinomas.

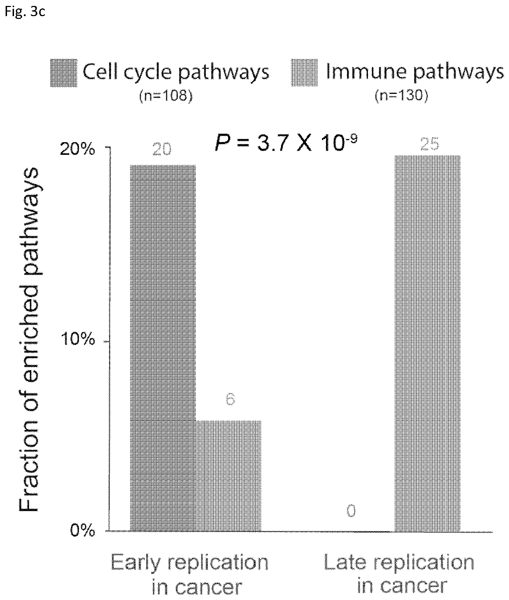

It may be inferred that the decrease in the expression level of the immune gene due to the decrease in global methylation as a result of Example 2 should be consistent with the above change in the late DNA-replicating region. In fact, as shown in FIG. 3C, it was statistically identified that the genes involved in cell division were more concentrated in the early DNA-replicating region, while the genes involved in the immune response were concentrated in the late DNA-replicating region.

**Example 4. Correlation between global DNA methylation and immunotherapy response**

4-1. Patient cohort for lung cancer checkpoint inhibitors

Sixty advanced non-small cell lung cancer patients treated with anti-PD-1/PD-L1 from 2014 to 2017 at Samsung Medical Center (SMC) were enrolled in this study.

Clinical response was assessed via follow-up for at least 6 months according to the response evaluation criteria of Solid Tumors (RECIST) version 1.1. Responses to immunotherapy were classified into durable clinical benefit (DCB, responder) or nondurable benefit (NDB, non-responder). Partial response (PR) or stable disease (SD) lasting 6 months or longer was considered as DCB/responder. Progressive disease (PD) or SD lasting less than 6 months was considered as an NDB/non-responder. Progression-free survival (PFS) was calculated from the start of treatment to an earlier one of the date of progression or death date. When the patient was alive without progression, the patient was censored on the date of the last follow-up for PFS. The methylation data for 81 samples of the same type of lung cancer, named as IDIBELL cohort were provided from a following literature. Davalos, V. et al. Epigenetic prediction of response to anti-PD-1 treatment in non-small-cell lung cancer: a multicentre, retrospective analysis. Lancet Respir. Med. 6, 771-781 (2018).

4-2. Analysis of methylation and tumor mutation burden on SMC samples

Tumor samples were obtained before anti-PD1/PD-L1 treatment. After formalin fixation, the tumor samples were embedded in paraffin or were stored in a fresh state. DNA was prepared using the AllPrep DNA/RNA Mini Kit (Qiagen, 80204), AllPrep DNA/RNA Micro Kit (Qiagen, 80284) or QIAamp DNA FFPE Tissue Kit (Qiagen, 56404) for the preparation of the library for whole exome sequencing. Library preparation was performed using SureSelectXT Human All Exon V5 (Agilent, 5190-6209) according to the instructions. The linked DNA was hybridized using whole exome baits of SureSelectXT Human All Exon V5, and then the library was quantified by Qubit and 2200 Tapestation. Then, the mated ends of $2 \times 100$ bp were sequenced on the Illumina HiSeq 2500 platform.

The target range of the normal sample was $50 \times$, and that of the tumor sample was $100 \times$. Strelka2 54 was used to derive somatic cell variant. Single nucleotide variants (SNVs) and indels covered by at least 10 and 5 reads were selected in the tumor, respectively. Further, the general germ cell variant present in dbSNP 150 was further filtered. ANNOVAR was used to annotate somatic cell variant.

Then, the methylation analysis was performed according to the guidelines of the Infinium Methylation EPIC BeadChIP Kit (Illumina, WG-317-1002). The raw methylation value was pretreated into a beta value, which was then processed as described in "Measurement of the global methylation level" of Example 1-2.

4-3. Evaluation of effect of global DNA methylation level on checkpoint inhibitors

The effect on the checkpoint inhibitors was evaluated in high and low groups based on the median of the global DNA methylation level measured via the above process.

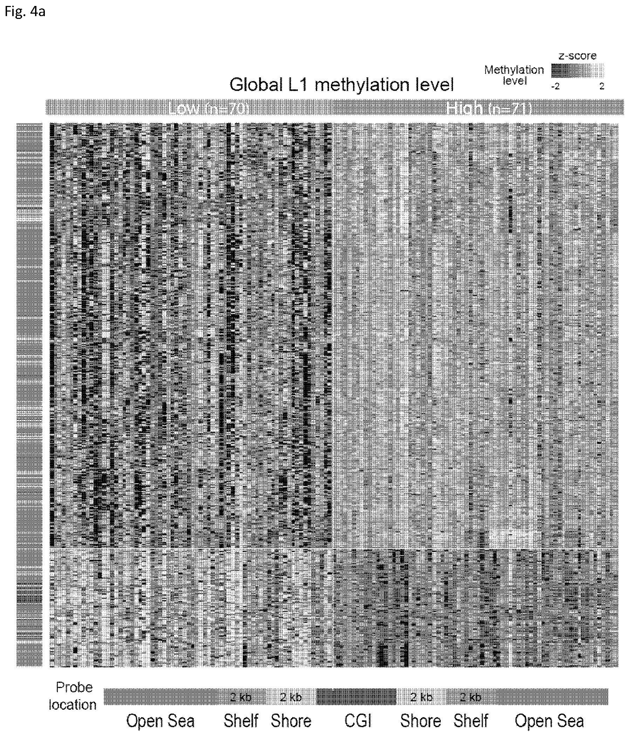

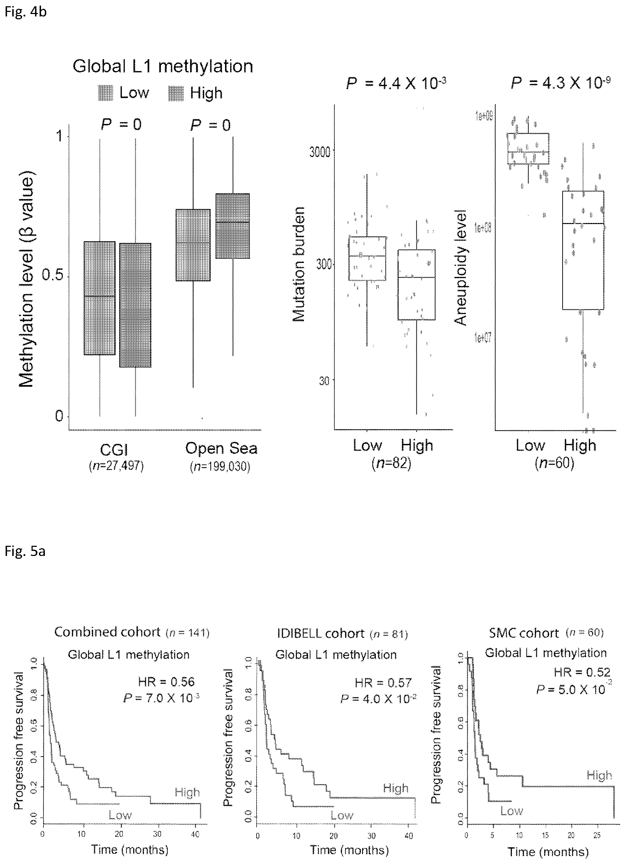

As a result, it was found that in the low group, hypomethylation occurred mainly in the open sea region, whereas hypermethylation was observed in the CpG island and the surrounding region (FIG. 4A and FIG. 4B). As observed in FIG. 1 of the TCGA example, it was identified that tumor mutation burden and chromosome aneuploidy were also increased in the low group.

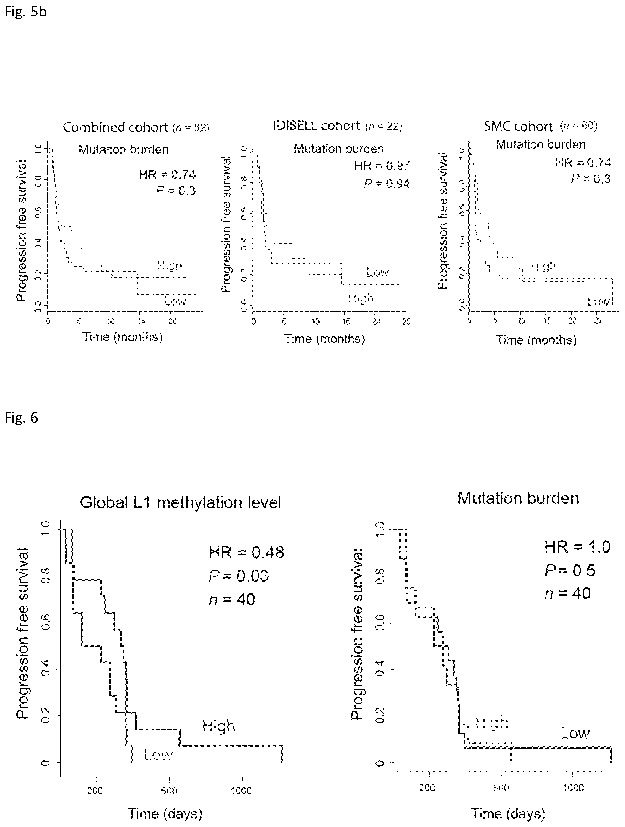

Importantly, it was found that the survival rate after checkpoint treatment was lower in the variant group. As may be seen from FIG. 5A and FIG. 5B, the tumor mutation burden did not have a significant correlation with the survival rate. However, it was identified that when the global DNA methylation was used as a prediction variable, the survival rate decreased significantly in the low group.

The above results indicate that the global DNA methylation level may be used as a significant marker indicating the response to immunotherapy of cancer patients and further predicting the prognosis. This suggests that the global DNA methylation level acts as a predictor having a higher accuracy related to the cancer treatment response than the previously known tumor mutation burden acts as.

**Example 5. Correlation between global DNA methylation and response to immunotherapy against melanoma**

In addition, progression-free survival data of 15 melanoma patients which have received treatment with immune checkpoint inhibitors (Ipilimumab, Yervoy, or Pembrolizumab) were obtained from Ock et.al (Nat. Commun. 8, 1050 (2017)). In addition, data of 25 patients who have received other types of immunotherapy were obtained from the GDC legacy archive (https://portal.gdc.cancer.gov/legacy-archive). Methylation and mutation data thereof were obtained from TCGA as in Example 1-1. The correlation between global DNA methylation level and the patient survival rate was evaluated in the same manner as described in Example 3 above.

As a result, as shown in FIG. 6, when the global DNA methylation level was low, the progression-free survival rate decreased. However, the tumor mutation burden had no significant correlation to the progression-free survival rate.

As described above, although the embodiments have been described based on the limited drawings, a person of ordinary skill in the art may apply various technical modifications and variations based on the above teachings. For example, appropriate results may be achieved although the described details are performed in a different order from that in the described method, and/or the described components are combined with each other in a form different from that of the described method, or are replaced or substituted with other components or equivalents.

Therefore, other implementations, other embodiments and claims and equivalents thereto fall within the scope of the following claims.

**Claims**

1. A method for providing information for predicting a response to cancer treatment, the method comprising:

    obtaining information on a global DNA methylation level detected from a sample of a cancer patient; and
    evaluating a response to cancer treatment based on the information on the global DNA methylation level.

2. The method of claim 1, wherein the global DNA methylation level is calculated as an average value of methylation levels occurring in a plurality of LINE-1 (Long Interspersed Nuclear Element-1) transposons.

3. The method of claim 1, wherein the LINE-1 transposon is L1HS or L1PA.

4. The method of claim 1, wherein the evaluating of the response to the cancer treatment includes determining that resistance to the cancer treatment is high when the global DNA methylation level is low.

5. The method of claim 1, wherein the cancer treatment is immunotherapy.

6. The method of claim 1, wherein the sample includes cancer tissue, whole blood, serum, saliva, sputum, cerebrospinal fluid, or urine.

7. The method of claim 1, wherein the cancer includes melanoma, bladder cancer, esophageal cancer, glioma, adrenal cancer, sarcoma, thyroid cancer, colorectal cancer, prostate cancer, head and neck cancer, urinary tract cancer, stomach cancer, pancreatic cancer, liver cancer, testicular cancer, ovarian cancer, endometrial cancer, cervical cancer, brain cancer, breast cancer, kidney cancer or lung cancer.

8. A method for providing information for predicting a response to cancer treatment, the method comprising:

   obtaining information on a global DNA methylation level detected from a sample of a cancer patient;
   obtaining information on a tumor mutation burden from the sample; and
   evaluating a response to cancer treatment based on the information on the global DNA methylation level and the information on the tumor mutation burden.

9. A method for providing information for predicting a response to cancer treatment, the method comprising:

   obtaining information on a global DNA methylation level detected from a sample of a cancer patient;
   obtaining information on a tumor mutation burden from the sample;
   obtaining information on chromosome aneuploidy from the sample; and
   evaluating a response to cancer treatment based on the information on the global DNA methylation level information, the information on the tumor mutation burden, and the information on the chromosome aneuploidy information.

10. A device for predicting a response to cancer treatment,
    wherein the device includes a processor configured to:

    obtain information on a global DNA methylation level detected from a sample of a patient; and
    evaluate a response to cancer treatment based on the information on the global DNA methylation level.

11. The device of claim 10, wherein the processor is further configured to determine that resistance to the cancer treatment is high when the global DNA methylation level is low.

12. The device of claim 10, wherein the cancer treatment is immunotherapy.

13. The device of claim 10, wherein the sample includes cancer tissue, whole blood, serum, saliva, sputum, cerebrospinal fluid, or urine.

14. The device of claim 10, wherein the cancer includes melanoma, bladder cancer, esophageal cancer, glioma, adrenal cancer, sarcoma, thyroid cancer, colorectal cancer, prostate cancer, head and neck cancer, urinary tract cancer, stomach cancer, pancreatic cancer, liver cancer, testicular cancer, ovarian cancer, endometrial cancer, cervical cancer, brain cancer, breast cancer, kidney cancer or lung cancer.

15. A device for predicting a response to cancer treatment,
    wherein the device includes a processor configured to:

    obtain information on a global DNA methylation level detected from a sample of a patient;
    obtain information on tumor mutation burden from the sample; and
    evaluate a response to cancer treatment based on the information on the global DNA methylation level and the information on the tumor mutation burden.

16. A device for predicting a response to cancer treatment,
    wherein the device includes a processor configured to:

    obtain information on a global DNA methylation level detected from a patient's sample;
    obtain information on tumor mutation burden from the sample;
    obtain information on chromosome aneuploidy from the sample; and
    evaluate a response to cancer treatment based on the information on the global DNA methylation level, the information on the tumor mutation burden, and the information on the chromosome aneuploidy.

FIG. 1

Fig. 2a

Fig. 2b

Predictor variables
(Purity, Age, and Stage adjusted)

Global methylation level

Mutation burden

Aneuploidy level

Tumor type

Hallmark
immune gene sets

ALLOGRAFT_REJECTION
COAGULATION
COMPLEMENT
IL2_STAT5_SIGNALING
IL6_JAK_STAT3_SIGNALING
INFLAMMATORY_RESPONSE
INTERFERON_ALPHA_RESPONSE
INTERFERON_GAMMA_RESPONSE
TNFA_SIGNALING_VIA_NFKB

MAJOR_HISTOCOMPATIBILITY_COMPLEX
ANTIGEN_PROCESSING_AND_PRESENTATION
CYTOKINE_CYTOKINE_RECEPTOR_INTERACTION

Hallmark
proliferation gene sets

E2F_TARGETS
G2M_CHECKPOINT
MITOTIC_SPINDLE
MYC_TARGETS_V1
MYC_TARGETS_V2
P53_PATHWAY

BLCA  BRCA  CESC  CRC  ESCA  HNSC  KIRC  KIRP  LGG  LIHC  LUAD  LUSC  PAAD  PCPG  PRAD  SARC
SKCM  STAD  TGCT  THCA  UCEC

NES

13

Fig. 3a

Fig. 3b

Fig. 3c

Fig. 4a

Global L1 methylation level

Methylation level — z-score (-2 to 2)

Low (n=70)    High (n=71)

Probe location

Open Sea    Shelf    Shore    CGI    Shore    Shelf    Open Sea

2 kb    2 kb    2 kb    2 kb

Fig. 4b

Global L1 methylation

Fig. 5a

Fig. 5b

Fig. 6

## INTERNATIONAL SEARCH REPORT

| International application No. |
|---|
| **PCT/KR2020/012487** |

**A.    CLASSIFICATION OF SUBJECT MATTER**

**C12Q 1/6886**(2018.01)i

According to International Patent Classification (IPC) or to both national classification and IPC

**B.    FIELDS SEARCHED**

Minimum documentation searched (classification system followed by classification symbols)

C12Q 1/6886(2018.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: DNA, 메틸화(methylation), 변이(aberration), 면역항암치료(anticancer immunotherapy), 반응성(response)

**C.    DOCUMENTS CONSIDERED TO BE RELEVANT**

| Category* | Citation of document, with indication, where appropriate, of the relevant passages | Relevant to claim No. |
|---|---|---|
| X<br><br>Y<br>A | CHATTERJEE, A. et al. Marked Global DNA Hypomethylation Is Associated with Constitutive PD-L1 Expression in Melanoma. iScience. 26 June 2018, vol. 4, pp. 312-325<br>    See abstract; Discussion; and page 313. | 1,4-7,10-14<br><br>8,9,15,16<br>2,3 |
| Y | CHAN, T. A. et al. Development of tumor mutation burden as an immunotherapy biomarker: utility for the oncology clinic. Annals of Oncology. electronic publication. 05 November 2018, vol. 30, no. 1, pp. 44-56<br>    See abstract. | 8,9,15,16 |
| Y | DAVOLI, T. et al. Tumor aneuploidy correlates with markers of immune evasion and with reduced response to immunotherapy. Science. 2017, vol. 355, document eaaf8399, pp. 1-14<br>    See page 1. | 9,16 |
| A | OHKA, F. et al. The Global DNA Methylation Surrogate LINE-1 Methylation Is Correlated with MGMT Promoter Methylation and Is a Better Prognostic Factor for Glioma. PLoS ONE. August 2011, vol. 6, no. 8, document e23332, pp. 1-10<br>    See entire document. | 1-16 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **31 December 2020** | **04 January 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2020/012487** |

**C.    DOCUMENTS CONSIDERED TO BE RELEVANT**

| Category* | Citation of document, with indication, where appropriate, of the relevant passages | Relevant to claim No. |
| --- | --- | --- |
| A | FOUAD, M. A. et al. Impact of Global DNA Methylation in Treatment Outcome of Colorectal Cancer Patients. Frontiers in Pharmacology. October 2018, vol. 9, document 1173, pp. 1-14<br>    See entire document. | 1-16 |
| A | KR 10-1539104 B1 (EWHA UNIVERSITY - INDUSTRY COLLABORATION FOUNDATION) 23 July 2015 (2015-07-23)<br>    See entire document. | 1-16 |
| PX | JUNG, H. et al. DNA methylation loss promotes immune evasion of tumours with high mutation and copy number load. Nature Communications. electronic publication. 19 September 2019, vol. 10, document 4278, pp. 1-12<br>    See entire document. | 1-16 |

Form PCT/ISA/210 (second sheet) (July 2019)

| INTERNATIONAL SEARCH REPORT | | International application No. | |
| --- | --- | --- | --- |
| Information on patent family members | | **PCT/KR2020/012487** | |

| Patent document cited in search report | Publication date (day/month/year) | Patent family member(s) | Publication date (day/month/year) |
| --- | --- | --- | --- |
| KR 10-1539104 B1 | 23 July 2015 | None | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190114977

**Non-patent literature cited in the description**

- *Semin Oncol,* 2011, vol. 38 (2), 173-85
- **MCCABE MT ; BRANDES JC ; VERTINO PM.** Cancer DNA methylation: molecular mechanisms and clinical implications. *Clin Cancer Res.,* 2009, vol. 15 (12), 3927-37
- **TAYLOR et al.** *Cancer Cell,* 2018, vol. 33, 676-689.e3
- *Immunity,* 2018, vol. 48, 812-830.e14
- **SUBRAMANIAN, A. et al.** *Proc. Natl. Acad. Sci.,* 2005, vol. 102, 15545-15550
- **DAVALOS, V. et al.** Epigenetic prediction of response to anti-PD-1 treatment in non-small-cell lung cancer: a multicentre, retrospective analysis. *Lancet Respir. Med.,* 2018, vol. 6, 771-781
- **OCK.** *Nat. Commun.,* 2017, vol. 8, 1050